# EUROPEAN PATENT APPLICATION

(11) **EP 3 473 672 A1**
(43) Date of publication of application: **24.04.2019**
(21) Application number: 17813422.7
(22) Date of filing: 16.06.2017
(51) Int. Cl.: C08L 21/00, C08K 5/3462, B60C 1/00

(54) **ADDITIVE FOR RUBBER**

(30) Priority: 16.06.2016 JP 2016120020
(71) Applicant: Bridgestone Corporation, Tokyo 104-8340 (JP); Kao Corporation, Chuo-ku Tokyo 103-8210 (JP)
(72) Inventor: FUJITA, Satomi, Wakayama-shi Wakayama 640-8580 (JP); TAKANO, Tetsuo, Wakayama-shi Wakayama 640-8580 (JP); HAYAKAWA, Kotaro, Tokyo 104-8340 (JP)
(74) Representative: Lang, Johannes
(86) International application number: PCT/JP2017/022275
(87) International publication number: WO 2017/217528

(57) **Abstract**

An additive for a rubber contains a compound represented by the following general formula (I) wherein R¹ is a divalent hydrocarbon group having 1 or more and 20 or less carbon atoms; R² and R³ are each independently a hydrogen atom or a hydrocarbon group having 1 or more and 6 or less carbon atoms; X is a hydrogen atom or a group represented by R⁴-COO-, R⁴-CONH- or R⁴-O-; and R⁴ is a hydrocarbon group having 1 or more and 20 or less carbon atoms.

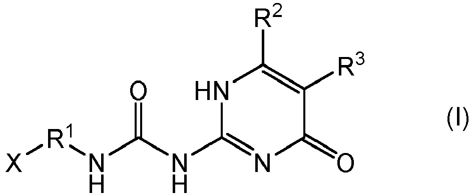

## Description

### Technical Field

The present invention relates to an additive for a rubber.

### Background Art

The term "rubber" refers to an amorphous soft polymer material, and particularly refers to a material including an organic polymer such as a natural rubber and a synthetic rubber as a main component, having a high limit of elasticity and a low elastic modulus, i.e., an elastic rubber, in many cases. Taking advantage of the properties, a composition containing a rubber (rubber composition) is used in various fields such as tires, sealing materials and seismic isolation or vibration damping materials.

For example, in a vehicle tire, the rubber elasticity of a rubber allows impacts generated when a vehicle travels on a road surface having irregularities to be absorbed, achieving riding comfort of the vehicle, or easing impacts on the vehicle itself. Further, water and air are unlikely to pass through a rubber, which allows a tire to tightly retain the air and to withstand rain and snow. Furthermore, due to a large frictional force of a rubber, a tire in contact with a road surface has a large frictional force, so that motive force and braking force can be quickly transmitted to a road surface, unlikely to cause slippage.

With effective use of such properties of a rubber, various additives for a rubber are used to obtain even more favorable performance.

Examples of the typical additives include sulfur. Addition of sulfur allows rubber molecules to be crosslinked to each other, so that the rubber distinctively exhibits properties of a rubber elastic body. The crosslinks through sulfur atoms allow rubber molecules to be connected to each other through covalent bonding, and are referred to as permanent crosslinks because the crosslinks are not easily broken.

On the other hand, hydrogen bond forming sites may be introduced into rubber molecular chains, so that the introduced sites are connected to each other through hydrogen bonding to form crosslinks. Such crosslinks allow rubber molecules to be connected to each other through hydrogen bonding, and are referred to as nonpermanent crosslinks because the crosslinks are easily detachable by switching of distortion input or temperature change. Examples of the method for introducing hydrogen bond forming sites into rubber molecular chains include a method for chemically modifying rubber molecular chains or a grafting method. Through coexistence of the different crosslinking methods, materials having different properties suitable for use can be obtained.

For example, PTL 1 discloses an elastomer material containing a flexible polymer chain including a combination of a permanent crosslinking bridge having a covalent bond and a crosslinking bridge having a non-covalent bond between chains, wherein the crosslinking bridge includes an association group based on a nitrogen-containing hetero ring.

PTL 2 discloses a polymer modified by grafting of a nitrogen-containing associating molecule including at least one unit which can be bonded to each other or to a filler through non-covalent bonding.

PTL 3 discloses a rubber composition based on at least one diene elastomer, a reinforcement filler, a chemical crosslinking agent and at least one specific modifier, which is particularly used in producing tires.

PTL4 discloses a polymer modified by grafting nitrogen-containing associating molecules along the polymer chain.

### Citation List

### Patent Literature

PTL1: National Publication of International Patent Application No. 2012-503060
PTL2: National Publication of International Patent Application No. 2013-530299
PTL3: National Publication of International Patent Application No. 2013-531726
PTL4: National Publication of International Patent Application No. 2013-531727

### Summary of Invention

### Technical Problem

Regarding rubber compositions for use in producing tires, for example, PTL 3 discloses that a rubber composition having mechanical properties including excellent stiffness and high elongation at break under a moderate deformation in combination with a low hysteresis is suitable for improving the balance between rolling resistance and resistance to a large deformation in producing tires.

However, in order to obtain a tire satisfying all of the fuel economy, the steering stability and the riding comfort of a vehicle, a technique for controlling the storage modulus corresponding to the magnitude of deformation of the tire along with reduction in the loss tangent (tan δ) is required.

An object of the present invention is to provide an additive for a rubber that allows various properties such as the storage modulus at a large deformation and at a small deformation and tan δ to be controlled when added to a rubber composition, and a rubber composition capable of satisfying all of the fuel economy, the steering stability and the riding comfort of a vehicle when used in a tire.

### Solution to Problem

The present inventors have found that addition of an additive for a rubber having a specific structure to a rubber composition can solve the problem.

Namely, the present invention relates to the following [1] to [7].
[1] An additive for a rubber, comprising a compound represented by the following general formula (I): 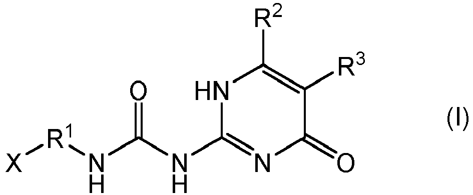 wherein R¹ is a divalent hydrocarbon group having 1 or more and 20 or less carbon atoms; R² and R³ are each independently a hydrogen atom or a hydrocarbon group having 1 or more and 6 or less carbon atoms; X is a hydrogen atom or a group represented by R⁴-COO-, R⁴-CONH- or R⁴-O-; and R⁴ is a hydrocarbon group having 1 or more and 20 or less carbon atoms.
[2] A rubber composition comprising the additive for a rubber according to item [1] and a rubber.
[3] A method for producing the rubber composition according to item [2], comprising a step of compounding and kneading a compound represented by the general formula (I).
[4] Use of a compound represented by the general formula (I) in producing the rubber composition according to item [2].
[5] A tire produced by using the rubber composition according to item [2].
[6] A method for producing the tire according to item [5], comprising a step of molding the rubber composition.
[7] Use of the rubber composition according to item [2] in a tire.

### Advantageous Effects of Invention

Addition of the additive for a rubber of the present invention to a rubber composition allows the storage modulus at a small deformation to be improved without excessive improvement in the storage modulus at a large deformation, with tan δ being reduced. With use of the rubber compound containing the additive for a rubber in a tire, all of the fuel economy, the steering stability and the riding comfort of a vehicle can be satisfied.

### Description of Embodiments

### [Additive for rubber]

The additive for a rubber of the present invention containes a compound represented by the following general formula (I): 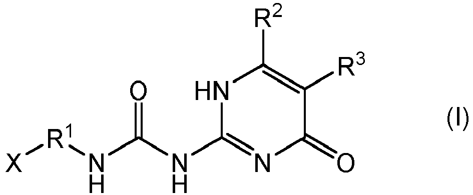 wherein R¹ is a divalent hydrocarbon group having 1 or more and 20 or less carbon atoms; R² and R³ are each independently a hydrogen atom or a hydrocarbon group having 1 or more and 6 or less carbon atoms; X is a hydrogen atom or a group represented by R⁴-COO-, R⁴⁻CONH- or R⁴-O-; and R⁴ is a hydrocarbon group having 1 or more and 20 or less carbon atoms.

Addition of the additive for a rubber of the present invention to a rubber composition allows the storage modulus at a small deformation to be improved without excessive improvement in the storage modulus at a large deformation, with tan δ being reduced. In the additive for a rubber of the present invention, the compounds represented by the general formula (I) may be used singly or in combinations of two or more.

The additive for a rubber and the rubber composition of the present invention are suitable for use in a tire. It is known that reduction in the rolling resistance of a tire is effective for saving fuel consumption of a vehicle, and reduction in tan δ of the rubber composition for use in a tire is effective for reducing the rolling resistance.

Also, enhanced block stiffness with a high storage modulus of a tire is generally effective for improving the steering stability of a vehicle. It is, however, known that when a rubber composition having an excessively high storage modulus at a large deformation is used in a tire, impacts in vehicle traveling cannot be absorbed, resulting in worsened riding comfort. In contrast, a rubber composition for use in a tire having a high storage modulus at a small deformation improves the block stiffness of the tire, and the steering stability of a vehicle can be secured.

As a result of research considering that when a rubber composition having an improved storage modulus at a small deformation without an excessively improved storage modulus at a large deformation, and a small tan δ, is used in a tire, all of the fuel economy, the steering stability and the riding comfort of a vehicle can be satisfied, an additive for a rubber capable of solving the problem has been found in the present invention.

Details of the action mechanism of the effect of the additive for a rubber of the present invention are presumed to be as follows, though a part thereof is unknown.

Through addition of the additive for a rubber of the present invention to a rubber composition, non-permanent crosslinks are formed among rubber molecule chains. When a strong force is applied to a rubber composition from the outside to cause a large deformation, the non-permanent crosslinks dissociate, so that the crosslink density decreases. In contrast, when a weak force is applied to a rubber composition from the outside to cause a small deformation, the non-permanent crosslinks do not dissociate, so that a high crosslink density can be maintained. It is therefore presumed that while the storage modulus at a large deformation is maintained or reduced without excessive improvement, the storage modulus at a small deformation can be improved. As a result, with use of the additive for a rubber of the present invention in a tire, the steering stability can be improved without impairing the riding comfort of a vehicle.

The non-permanent crosslinks formed by addition of the additive for a rubber of the present invention to a rubber composition have more crosslinking points formed by hydrogen bonding than conventional non-permanent crosslinks, so that strong bonding is formed to reduce tan δ. As a result, when the additive for a rubber is used in a tire, rolling resistance can be reduced, and it is therefore presumed that fuel economy can be improved. The explanation of the present disclosure may be, however, not limited to the mechanism.

In introduction of non-permanent crosslinks, conventionally, it has been a general practice to newly add a step of grafting nitrogen-containing associating molecules to a rubber component prior to a step of kneading (two-stage process). The additive for a rubber of the present invention, however, allows a non-permanent crosslinking structure to be introduced into a rubber composition in a single kneading step without a step of grafting, so that tan δ and the storage modulus corresponding to the magnitude of deformation can be adjusted within desired ranges.

### <Compound represented by general formula (I)>

In the general formula (I), R¹ is a divalent hydrocarbon group having 1 or more and 20 or less carbon atoms. From the viewpoint of compatibility with a rubber and from the viewpoint of increasing the storage modulus at a small deformation without excessive improvement in the storage modulus at a large deformation and reducing tan δ, R¹ has preferably 2 or more carbon atoms. On the other hand, from the viewpoint of increasing the number of non-permanent crosslinking points per weight of a compound represented by the general formula (I), R¹ has preferably 18 or less carbon atoms, more preferably 12 or less carbon atoms, further preferably 8 or less carbon atoms, furthermore preferably 6 or less carbon atoms.

The divalent hydrocarbon group in R¹ may have any of a straight chain structure, a branched chain structure and a cyclic structure, or a combination thereof. Examples of the hydrocarbon group include an alkylene group, an alkenylene group, an alkylidene group and an arylene group. From the viewpoint of compatibility with a rubber, R¹ is preferably at least one selected from the group consisting of a straight chain or branched chain alkylene group and a straight chain or branched chain alkenylene group, more preferably at least one selected from the group consisting of a straight chain alkylene group and a straight chain alkenylene group. From the viewpoints of compatibility and reactivity with a rubber, a straight chain alkenylene group is more preferred.

Namely, in the general formula (I), R¹ is preferably at least one selected from the group consisting of a straight chain or branched chain alkylene group and a straight chain or branched chain alkenylene group having 1 or more and 20 or less carbon atoms, more preferably a straight chain alkenylene group having 1 or more and 20 or less carbon atoms. R¹ has preferably 2 or more carbon atoms and preferably 18 or less carbon atoms, more preferably 12 or less carbon atoms, further preferably 8 or less carbon atoms, furthermore preferably 6 or less carbon atoms.

In the general formula (I), R² and R³ are each independently a hydrogen atom or a hydrocarbon group having 1 or more and 6 or less carbon atoms. The hydrocarbon group has preferably 1 or more and 3 or less carbon atoms, more preferably 1 or 2 carbon atoms. The hydrocarbon group may have any of a straight chain structure, a branched chain structure and a cyclic structure, or a combination thereof. Examples of the hydrocarbon group include an alkyl group, an alkenyl group, an aryl group and an aralkyl group.

From the viewpoint of compatibility with a rubber and from the viewpoint of increasing the storage modulus at a small deformation without excessive improvement in the storage modulus at a large deformation and reducing tan δ, R² and R³ are each independently preferably a hydrogen atom or an alkyl group having 1 or more and 6 or less carbon atoms, more preferably a hydrogen atom or an alkyl group having 1 or more and 3 or less carbon atoms. Furthermore, from the viewpoints described above, preferably R² is an alkyl group having 1 or more and 6 or less carbon atoms and R³ is a hydrogen atom, more preferably R² is an alkyl group having 1 or more and 3 or less carbon atoms and R³ is an hydrogen atom, further preferably R² is an alkyl group having 1 or 2 carbon atoms and R³ is a hydrogen atom, furthermore preferably R² is a methyl group and R³ is a hydrogen atom.

In the general formula (I), X is a hydrogen atom or a group represented by R⁴-COO-, R⁴-CONH- or R⁴-O-, and R⁴ is a hydrocarbon group having 1 or more and 20 or less carbon atoms. From the viewpoint of the compatibility with a rubber, X is preferably a hydrogen atom or a group represented by R⁴-COO-, more preferably a group represented by R⁴-COO-.

From the viewpoint of the compatibility with a rubber and the viewpoint of increasing the storage modulus at a small deformation without excessive improvement in the storage modulus at a large deformation and reducing tan δ, R⁴ has preferably 2 or more carbon atoms, more preferably 3 or more carbon atoms. On the other hand, from the viewpoint of increasing the number of non-permanent crosslinking points per weight of a compound represented by the general formula (I), R⁴ has preferably 18 or less carbon atoms, more preferably 12 or less carbon atoms, further preferably 8 or less carbon atoms, furthermore preferably 6 or less carbon atoms.

The hydrocarbon group in R⁴ may have any of a straight chain structure, a branched chain structure and a cyclic structure, or a combination thereof. Examples of the hydrocarbon group include an alkyl group, an alkenyl group, an aryl group and an aralkyl group.

From the viewpoint of providing reactivity with a rubber to an additive for a rubber, R⁴ is preferably a hydrocarbon group having 2 or more and 20 or less carbon atoms, having one or more polymerizable unsaturated bonds, more preferably a hydrocarbon group having 2 or more and 20 or less carbon atoms, having a polymerizable unsaturated bond at an α position, further preferably an alkenyl group having 2 or more and 20 or less carbon atoms, having a polymerizable unsaturated bond at an α position, furthermore preferably an alkenyl group having 2 or 3 carbon atoms, having a polymerizable unsaturated bond at an α position, furthermore preferably an isopropenyl group.

In the present specification, the polymerizable unsaturated bond means an unsaturated bond capable of addition polymerization.

As the compound represented by the general formula (I), in particular, at least one selected from the group consisting of a compound represented by the following formula (I-I) and a compound represented by the following formula (I-II) is preferred, and a compound represented by the following formula (I-I) is more preferred.

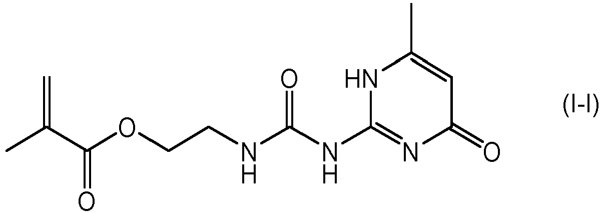

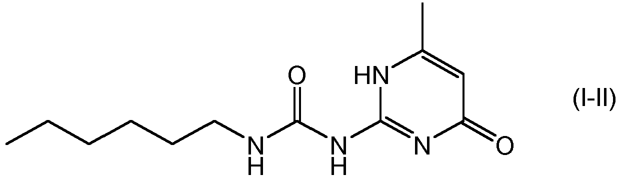

The method for producing a compound represented by the general formula (I) is not particularly limited and a known method can be used. For example, the production can be achieved by a reaction of a compound represented by the following general formula (1) (hereinafter also referred to as "raw material isocytosine") with a compound represented by the following general formula (2) (hereinafter also referred to as "raw material isocyanate") under heating conditions. 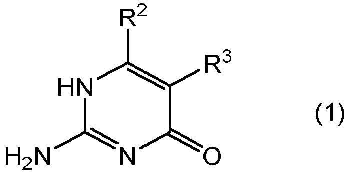 wherein R² and R³ are the same as above.

X-R¹-NCO (2)

wherein, R¹ and X are the same as above.

The reaction of the raw material isocytosine and the raw material isocyanate can be performed in the presence of a solvent. Although the solvent is not particularly limited as long as it can dissolve the raw material isocytosine and the raw material isocyanate, for example, dimethyl sulfoxide and dimethylformamide are preferred.

The raw material isocytosine and the raw material isocyanate may be fed into a reactor in advance to be heated, or the raw material isocytosine and a solvent may be fed into a reactor in advance to be heated and then the raw material isocyanate may be dropped thereto.

From the viewpoints of productivity and suppression of decomposition of the raw material, the reaction temperature is preferably 90 to 180°C, more preferably 120 to 160°C. From the viewpoints of productivity and reaction conversion, the reaction time is preferably 10 to 240 minutes, more preferably 20 to 120 minutes, which is different depending on the reaction temperature and the reaction scale.

The additive for a rubber of the present invention contains a compound represented by the general formula (I). From the viewpoint of obtaining the effect of the present invention, the content thereof is preferably 50 mass% or more, more preferably 70 mass% or more, further preferably 80 mass% or more, furthermore preferably 90 mass% or more. The upper limit of the content of the compound represented by the general formula (I) in an additive for a rubber is 100 mass%. Namely, the compound represented by the general formula (I) may be directly used as an additive for a rubber. The content of the compound represented by the general formula (I) in an additive for a rubber is preferably 100 mass%.

The additive for a rubber of the present invention may contain, for example, a solvent and water other than the compound represented by the general formula (I), within a range not impairing the object of the present invention.

The compound represented by the general formula (I) can be used as the additive for a rubber. Also, the compound can be used in the following rubber composition and in production of the rubber composition. The effect of the present invention can be thereby obtained.

### [Rubber composition]

The rubber composition of the present invention contains the additive for a rubber of the present invention and a rubber. The rubber composition of the present invention may further contain a reinforcement filler.

From the viewpoint of increasing the storage modulus at a small deformation without excessive improvement in the storage modulus at a large deformation and reducing tan δ, the content of the additive for a rubber in the rubber composition of the present invention is preferably 0.1 parts by mass or more, more preferably 0.5 parts by mass or more, further preferably 1 part by mass or more, furthermore preferably 2 parts by mass or more relative to 100 parts by mass of the rubber described below. Also, from the viewpoint of breaking strength of a rubber composition, the content of the additive for a rubber in the rubber composition is preferably 20 parts by mass or less, more preferably 15 parts by mass or less, further preferably 10 parts by mass or less, furthermore preferably 8 parts by mass or less, furthermore preferably 6 parts by mass or less relative to 100 parts by mass of a rubber.

### <Rubber>

Examples of the rubber for use in the rubber composition of the present invention include a diene rubber.

Examples of the diene rubber include at least one selected from the group consisting of a natural rubber (NR) and a synthetic diene rubber.

Examples of the specific synthetic diene rubber include a polybutadiene rubber (BR), a synthetic polyisoprene rubber (IR), a styrene-butadiene copolymer rubber (SBR), a styrene-isoprene copolymer rubber (SIR), an ethylene-butadiene copolymer rubber, a propylene-butadiene copolymer rubber, an ethylenepropylene-butadiene copolymer rubber, an ethylene-α-olefin-diene copolymer rubber, a butyl rubber, a halogenated butyl rubber, a copolymer of styrene and isobutylene having a halogenated methyl group, a chloroprene rubber, and an acrylonitrile-butadiene copolymer rubber.

Preferably, the diene rubber contains a styrene-butadiene copolymer rubber, from the viewpoint of reducing the rolling resistance when used in a tire, with reduction in tan δ of the rubber composition. From the viewpoint described above, the content of the styrene-butadiene copolymer rubber in a diene rubber is preferably 50 mass% or more, more preferably 70 mass% or more, further preferably 80 mass% or more, furthermore preferably 90 mass% or more. The upper limit of the content is 100 mass%.

These diene rubbers may be used singly or in combinations of two or more. Also, the diene rubber for use may be modified or may be unmodified.

From the viewpoint of exhibiting properties derived from a rubber, the rubber content of the rubber composition is preferably 30 mass% or more, more preferably 40 mass% or more, further preferably 50 mass% or more. Also, the rubber content of the rubber composition is preferably 99.9 mass% or less, more preferably 99.5 mass% or less, further preferably 99 mass% or less, furthermore preferably 98 mass% or less, furthermore preferably 94 mass% or less, furthermore preferably 92 mass% or less, furthermore preferably 90 mass% or less, furthermore preferably 85 mass% or less, furthermore preferably 80 mass% or less, furthermore preferably 70 mass% or less.

### <Reinforcement filler>

Preferably, the rubber composition of the present invention further contains a reinforcement filler from the viewpoints of reinforcing the mechanical properties of the rubber and obtaining a rubber composition exhibiting a desired storage modulus and tan δ.

Examples of the reinforcement filler for use in the rubber composition of the present invention include a carbon black, and an organic reinforcement filler and an inorganic reinforcement filler which are described below. These reinforcement fillers may be used singly or in combinations of two or more.

As the carbon black, a known carbon black of which the ranges of the amount of I₂ adsorbed, the CTAB specific surface area, the amount of N₂ adsorbed, and the amount of DBP adsorbed are appropriately selected may be used, as long as it improves the mechanical properties and the like. The carbon black is not particularly limited, and examples include carbon blacks having a grade of GPF, FEF, HAF, ISAF or SAF. These carbon blacks may be used singly or in combinations of two or more.

Examples of the organic reinforcement filler include an organic-functionalized polyvinyl aromatic filler described in WO2006/069792 and WO2006/069793.

Examples of the inorganic reinforcement filler include at least one selected from the group consisting of silica, aluminum hydroxide, clay, talc, calcium carbonate and zeolite. From the viewpoint of obtaining a rubber composition exhibiting an excellent storage modulus and tan δ, the inorganic reinforcement filler is preferably at least one selected from the group consisting of silica and aluminum hydroxide, more preferably silica.

The silica is not particularly limited, and examples thereof include wet silica (hydrous silicic acid), dry silica (anhydrous silicic acid), calcium silicate and aluminum silicate. Among them, from the viewpoint of availability, wet silica is preferred. These silicas may be used singly or in combinations of two or more.

From the viewpoint of reinforcement, the content of the reinforcement filler in a rubber composition is preferably 1 part by mass or more, more preferably 5 parts by mass or more, further preferably 10 parts by mass or more, furthermore preferably 20 parts by mass or more, furthermore preferably 30 parts by mass or more, furthermore preferably 40 parts by mass or more relative to 100 parts by mass of a rubber. Also, from the viewpoint of processability of a rubber composition, the content of the reinforcement filler in the rubber composition is preferably 120 parts by mass or less, more preferably 100 parts by mass or less, further preferably 80 parts by mass or less relative to 100 parts by mass of a rubber.

### <Coupling agent>

In the case where a rubber composition of the present invention contains an inorganic reinforcement filler, preferably the rubber composition further contains a coupling agent to increase the compounding effect of the inorganic reinforcement filler. Although the coupling agent is not particularly limited, from the viewpoint of the reactivity with an inorganic reinforcement filler, a silane coupling agent is preferred, and a sulfur atom-containing silane coupling agent is more preferred.

Examples of the sulfur atom-containing silane coupling agent include bis(3-triethoxysilylpropyl)tetrasulfide, bis(3-triethoxysilylpropyl)trisulfide, bis(3-triethoxysilylpropyl)disulfide, bis(2-triethoxysilylethyl)tetrasulfide, bis(3-trimethoxysilylpropyl)tetrasulfide, bis(2-trimethoxysilylethyl)tetrasulfide, 3-mercaptopropyl trimethoxysilane, 3-mercaptopropyl triethoxysilane, 2-mercaptoethyl trimethoxysilane, 2-mercaptoethyl triethoxysilane, 3-trimethoxysilylpropyl-N,N-dimethylthiocarbamoyl tetrasulfide, 3-triethoxysilylpropyl-N,N-dimethylthiocarbamoyl tetrasulfide, 2-triethoxysilylethyl-N,N-dimethylthiocarbamoyl tetrasulfide, 3-trimethoxysilylpropyl benzothiazolyl tetrasulfide, 3-triethoxysilylpropyl benzothiazolyl tetrasulfide, 3-triethoxysilylpropyl methacrylate monosulfide, 3-trimethoxysilylpropyl methacrylate monosulfide, bis(3-diethoxymethylsilylpropyl)tetrasulfide, 3-mercaptopropyl dimethoxymethyl silane, dimethoxymethylsilylpropyl-N,N-dimethyl thiocarbamoyl tetrasulfide and dimethoxymethylsilylpropyl benzothiazolyl tetrasulfide. These coupling agents may be used singly or in combinations of two or more.

Among them, bis(3-triethoxysilylpropyl)tetrasulfide is preferred.

From the viewpoint of reinforcement, the amount of a coupling agent compounded in a rubber composition is preferably 1 part by mass or more, more preferably 3 parts by mass or more, further preferably 5 parts by mass or more, relative to 100 parts by mass of an inorganic reinforcement filler. Also, from the viewpoint of reducing components not contributing to a coupling reaction, the amount is preferably 25 parts by mass or less, more preferably 20 parts by mass or less, further preferably 15 parts by mass or less.

### <Other components>

Other than the additive for a rubber, the rubber, the reinforcement filler and the coupling agent (with use of an inorganic reinforcement filler), a compounding agent conventionally used in a rubber composition including, for example, a vulcanizing agent such as sulfur; vulcanization accelerator such as zinc oxide, N-t-butyl-1,2-benzothiazolyl sulfenamide (TBBS), N-cyclohexyl-2-benzothiazyl sulfenamide (CBS), mercaptobenzothiazole (MBT), dibenzothiazyl disulfide (MBTS), 1,3-diphenyl guanidine (DPG), and a thiuram compound such as tetrakis(2-ethylhexyl)thiuram disulfide (TOT); a softening agent; stearic acid; and antioxidant may be appropriately selected to be compounded into the rubber composition of the present invention, within a range not impairing the object of the present invention. As these components, commercially available products may be suitably used.

### <Method for producing rubber composition>

The method for producing a rubber composition of the present invention is not particularly limited. For example, using a kneading machine such as a Banbury mixer, a roll and an intensive mixer, the components to be contained in a rubber composition can be compounded and mixed. From the viewpoint of obtaining the effect of the present invention, preferably the method for producing the rubber composition of the present invention contains a step of compounding and kneading a compound represented by the general formula (I).

More specifically, from the viewpoints of suppressing the occurrence of vulcanization during production of the rubber composition and improving the handling properties during production, the rubber composition is preferably produced by a method in which components other than a vulcanizing agent and a vulcanization accelerator among the components to be contained in the rubber composition are compounded and kneaded in advance (first kneading step), and subsequently the vulcanizing agent and the vulcanization accelerator are compounded and kneaded (second kneading step). According to the method, even when the first kneading step is performed under high temperature conditions, no vulcanization occurs, so that the rubber composition of the present invention can be produced at a high productivity.

From the viewpoint of suppressing thermal decomposition of the components, the maximum kneading temperature in the first kneading step is in the range of preferably 250°C or less, more preferably 200°C or less, further preferably 180°C or less. Also from the viewpoint of productivity, the kneading temperature in the first kneading step is preferably 100°C or more, more preferably 120°C or more, further preferably 140°C or more.

From the viewpoint of suppressing the occurrence of vulcanization during mixing, the maximum kneading temperature in the second kneading step is in the range of preferably 150°C or less, more preferably 130°C or less. Also from the viewpoint of productivity, the kneading temperature in the second kneading step is preferably 80°C or more, more preferably 100°C or more.

The rubber composition of the present invention can exhibit the effect particularly when used in a tire. The rubber composition can be used, for example, in a tread part of a tire.

### <Tire>

A tire of the present invention is produced by using the rubber composition of the present invention. Namely, the rubber composition of the present invention can be used in a tire or a component of a tire. Examples of the component of a tire in which the rubber composition can be suitably used include a tread and a tread base. More preferably, the tire of the present invention is produced by using the rubber composition in a tread part.

A method for producing the tire of the present invention is not particularly limited as long as it contains a step of molding the rubber composition. For example, a pneumatic tire is produced by a conventional method with use of the rubber composition of the present invention. Namely, at a stage where the rubber composition of the present invention is not vulcanized yet, for example, the rubber composition is extruded to form a component for use as a tread, which is then affixed on a tire molding machine by a conventional method to mold a raw tire. The raw tire is heated and compressed in a vulcanization machine to produce a tire.

Regarding the embodiments described above, the present invention discloses an additive for a rubber, a rubber composition, a method for producing the rubber composition, use of the rubber composition, a tire, a method for producing a compound, and use of the compound.
<1> An additive for a rubber, comprising a compound represented by the following general formula (I): 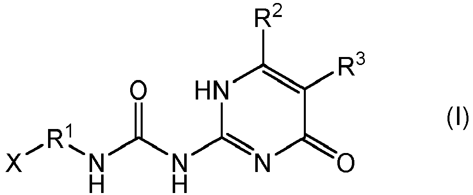 wherein R¹ is a divalent hydrocarbon group having 1 or more carbon atoms, preferably 2 or more carbon atoms, and having 20 or less carbon atoms, preferably 18 or less carbon atoms, more preferably 12 or less carbon atoms, further preferably 8 or less carbon atoms, furthermore preferably 6 or less carbon atoms; R² and R³ are each independently a hydrogen atom or a hydrocarbon group having 1 or more and 6 or less carbon atoms; X is a hydrogen atom or a group represented by R⁴-COO-, R⁴-CONH- or R⁴-O-; and R⁴ is a hydrocarbon group having 1 or more and 20 or less carbon atoms.
<2> The additive for a rubber according to item <1>, wherein in the general formula (I), the divalent hydrocarbon group in R¹ is at least one selected from the group consisting of an alkylene group, an alkenylene group, an alkylidene group and an arylene group, preferably at least one selected from the group consisting of a straight chain or branched chain alkylene group and a straight chain or branched chain alkenylene group, more preferably at least one selected from the group consisting of a straight chain alkylene group and a straight chain alkenylene group, further preferably a straight chain alkenylene group.
<3> The additive for a rubber according to item <1> or <2>, wherein in the general formula (I), R¹ is at least one selected from the group consisting of a straight chain alkylene group and a straight chain alkenylene group having 1 or more and 20 or less carbon atoms, more preferably a straight chain alkenylene group having 1 or more and 20 or less carbon atoms.
<4> The additive for a rubber according to any one of items <1> to <3>, wherein in the general formula (I), R² and R³ are each independently a hydrogen atom or an hydrocarbon group having 1 or more and 6 or less carbon atoms, preferably 1 or more and 3 or less carbon atoms, more preferably 1 or 2 carbon atoms.
<5> The additive for a rubber according to any one of items <1> to <4>, wherein in the general formula (I), the hydrocarbon group in R² and R³ each is an alkyl group, an alkenyl group, an aryl group or an aralkyl group.
<6> The additive for a rubber according to any one of items <1> to <5>, wherein in the general formula (I), R² is an alkyl group having 1 or more and 6 or less carbon atoms and R³ is a hydrogen atom, preferably R² is an alkyl group having 1 or more and 3 or less carbon atoms and R³ is a hydrogen atom, more preferably R² is an alkyl group having 1 or 2 carbon atoms and R³ is a hydrogen atom, further preferably R² is a methyl group and R³ is a hydrogen atom.
<7> The additive for a rubber according to any one of items <1> to <6>, wherein in the general formula (I), X is a hydrogen atom or a group represented by R⁴-COO-, preferably a group represented by R⁴-COO-.
<8> The additive for a rubber according to any one of items <1> to <7>, wherein R⁴ is a hydrocarbon group having 1 or more carbon atoms, preferably 2 or more carbon atoms, more preferably 3 or more carbon atoms, and 18 or less carbon atoms, preferably 12 or less carbon atoms, more preferably 8 or less carbon atoms, further preferably 6 or less carbon atoms.
<9> The additive for a rubber according to any one of items <1> to <8>, wherein the hydrocarbon group in R⁴ is an alkyl group, an alkenyl group, an aryl group or an aralkyl group.
<10> The additive for a rubber according to any one of items <1> to <9>, wherein R⁴ is a hydrocarbon group having 2 or more and 20 or less carbon atoms, having one or more polymerizable unsaturated bonds, preferably a hydrocarbon group having 2 or more and 20 or less carbon atoms, having a polymerizable unsaturated bond at an α position, more preferably an alkenyl group having 2 or more and 20 or less carbon atoms, having a polymerizable unsaturated bond at an α position, further preferably an alkenyl group having 2 or 3 carbon atoms, having a polymerizable unsaturated bond at an α position, furthermore preferably an isopropenyl group.
<11> The additive for a rubber according to any one of items <1> to <10>, wherein the compound represented by the general formula (I) is at least one selected from the group consisting of a compound represented by the following formula (I-I) and a compound represented by the following formula (I-II), preferably a compound represented by the following formula (I-I). 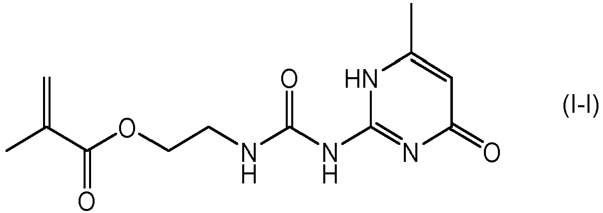 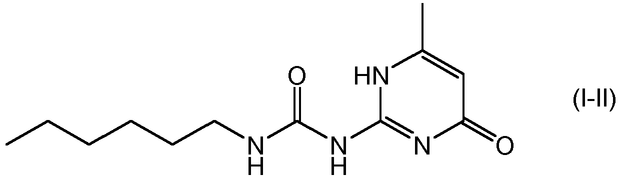
<12> The additive for a rubber according to any one of items <1> to <11>, wherein the content of the compound represented by the general formula (I) is preferably 50 mass% or more, more preferably 70 mass% or more, further preferably 80 mass% or more, furthermore preferably 90 mass% or more, and 100 mass% or less.
<13> A rubber composition comprising the additive for a rubber according to any one of items <1> to <12> and a rubber.
<14> The rubber composition according to item <13>, further comprising a reinforcement filler.
<15> The rubber composition according to item <13> or <14>, wherein the rubber is a diene rubber, preferably at least one selected from the group consisting of a natural rubber (NR) and a synthetic diene rubber.
<16> The rubber composition according to item <15>, wherein the synthetic diene rubber is at least one selected from the group consisting of a polybutadiene rubber (BR), a synthetic polyisoprene rubber (IR), a styrene-butadiene copolymer rubber (SBR), a styrene-isoprene copolymer rubber (SIR), an ethylene-butadiene copolymer rubber, a propylene-butadiene copolymer rubber, an ethylenepropylene-butadiene copolymer rubber, an ethylene-α-olefin-diene copolymer rubber, a butyl rubber, a halogenated butyl rubber, a copolymer of styrene and isobutylene having a halogenated methyl group, a chloroprene rubber, and an acrylonitrile-butadiene copolymer rubber.
<17> The rubber composition according to item <15> or <16>, wherein the diene rubber contains a styrene-butadiene copolymer rubber.
<18> The rubber composition according to item <17>, wherein the content of the styrene-butadiene copolymer rubber in the diene rubber is preferably 50 mass% or more, more preferably 70 mass% or more, further preferably 80 mass% or more, furthermore preferably 90 mass% or more, and 100 mass% or less.
<19> The rubber composition according to any one of items <13> to <18>, wherein the rubber content of the rubber composition is preferably 30 mass% or more, more preferably 40 mass% or more, further preferably 50 mass% or more, and preferably 99.9 mass% or less, more preferably 99.5 mass% or less, further preferably 99 mass% or less, furthermore preferably 98 mass% or less, furthermore preferably 94 mass% or less, furthermore preferably 92 mass% or less, furthermore preferably 90 mass% or less, furthermore preferably 85 mass% or less, furthermore preferably 80 mass% or less, furthermore preferably 70 mass% or less.
<20> The rubber composition according to any one of items <13> to <19>, wherein the content of the additive for a rubber in the rubber composition is preferably 0.1 parts by mass or more, more preferably 0.5 parts by mass or more, further preferably 1 part by mass or more, furthermore preferably 2 parts by mass or more, and preferably 20 parts by mass or less, more preferably 15 parts by mass or less, further preferably 10 parts by mass or less, furthermore preferably 8 parts by mass or less, furthermore preferably 6 parts by mass or less, relative to 100 parts by mass of a rubber.
<21> The rubber composition according to any one of items <14> to <20>, wherein the reinforcement filler is at least one selected from the group consisting of a carbon black, an organic reinforcement filler and an inorganic reinforcement filler.
<22> The rubber composition according to item <21>, wherein the inorganic reinforcement filler is at least one selected from the group consisting of silica, aluminum hydroxide, clay, talc, calcium carbonate and zeolite, preferably at least one selected from the group consisting of silica and aluminum hydroxide, more preferably silica.
<23> The rubber composition according to any one of items <14> to <22>, wherein the content of the reinforcement filler in the rubber composition is 1 part by mass or more, preferably 5 parts by mass or more, more preferably 10 parts by mass or more, further preferably 20 parts by mass or more, furthermore preferably 30 parts by mass or more, furthermore preferably 40 parts by mass or more, and 120 parts by mass or less, preferably 100 parts by mass or less, more preferably 80 parts by mass or less, relative to 100 parts by mass of a rubber.
<24> The rubber composition according to any one of items <21> to <23>, wherein the rubber composition contains an inorganic reinforcement filler and further a coupling agent, preferably a silane coupling agent, more preferably a sulfur atom-containing silane coupling agent.
<25> The rubber composition according to item <24>, wherein the amount of the coupling agent compounded in the rubber composition is 1 part by mass or more, preferably 3 parts by mass or more, more preferably 5 parts by mass or more, and 25 parts by mass or less, preferably 20 parts by mass or less, more preferably 15 parts by mass or less, relative to 100 parts by mass of the inorganic reinforcement filler.
<26> A method for producing the rubber composition according to any one of items <13> to <25>, comprising a step of compounding and kneading a compound represented by the general formula (I).
<27> Use of a compound represented by the general formula (I) in producing the rubber composition according to any one of items <13> to <25>.
<28> A tire produced by using the rubber composition according to any one of items <13> to <25>.
<29> The tire according to item <28>, wherein the rubber composition is used in a tread part.
<30> A method for producing the tire according to item <28> or <29>, comprising a step of molding the rubber composition.
<31> Use of the rubber composition according to any one of items <13> to <25> in a tire.
<32> Use of the rubber composition according to any one of items <13> to <25> in a tread part of a tire.
<33> A method for producing a compound represented by the general formula (I) comprising a step of reacting a compound represented by the following general formula (1) with a compound represented by the following general formula (2) under heating conditions: 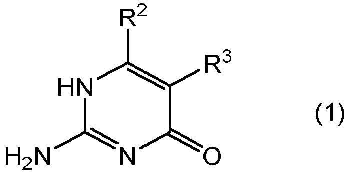 wherein R² and R³ are the same as described above, and

   X-R¹-NCO (2)

   wherein R¹ and X are the same as described above.
<34> Use of a compound represented by the general formula (I) as an additive for a rubber.

### Examples

Hereinafter, the present invention will be described with reference to Examples, though the present invention is not limited to the scope of the Examples. In the present Examples, the analysis of an additive for a rubber and the measurement and evaluation of a rubber composition were performed by the following methods.

### <¹H-NMR measurement>

The structural analysis of additives 1 and 2 for a rubber (a compound (I-I) and a compound (I-II) described below) obtained in the Examples was performed by ¹H-NMR measurement.

Each of the additives for a rubber dissolved in 0.05 v/v% TMS-containing heavy chloroform was subjected to ¹H-NMR measurement using a nuclear magnetic resonance apparatus (Agilent 400-MR DD2 system, manufactured by Agilent Technologies, Inc.). The chemical shift value of each peak (δ1H) was referenced to a TMS signal (0.00 ppm).

### <Measurement of storage modulus and tan δ>

The storage modulus and tan δ of a vulcanized rubber composition obtained in each example were measured using a rheometer "ARES-G2" (manufactured by TA Instruments - Waters LLC).

### (Storage modulus at small deformation)

The storage modulus measured at a temperature of 50°C, a dynamic strain of 0.1%, and a frequency of 10 Hz was shown as an index relative to 100 of the storage modulus in Comparative Example 2-1 in Table 1, and as an index relative to 100 of the storage modulus in Comparative Example 2-3 in Table 2. It indicates that as the index value increases, the storage modulus at a small deformation increases to have higher block stiffness, resulting in better steering stability of a vehicle when the rubber composition is used in a tire.

### (Storage modulus at large deformation)

The storage modulus measured at a temperature of 50°C, a dynamic strain of 10%, and a frequency of 10 Hz was shown as an index relative to 100 of the storage modulus in Comparative Example 2-1 in Table 1, and as an index relative to 100 of the storage modulus in Comparative Example 2-3 in Table 2. It indicates that as the index value increases, the storage modulus at a large deformation increases, so that when the rubber composition is used in a tire, impacts in vehicle traveling cannot be absorbed, resulting in worsened riding comfort.

### (tan δ)

The tan δ measured at a temperature of 50°C, a dynamic strain of 0.1%, and a frequency of 10 Hz was shown as an index relative to 100 of the tan δ in Comparative Example 2-1 in Table 1, and as an index relative to 100 of the tan δ of Comparative Example 2-3 in Table 2. It indicates that as the index value decreases, the rolling resistance of a tire decreases when the rubber composition is used in a tire, resulting in better fuel economy.

### Example 1-1 (Production of additive 1 for rubber)

18.8 g (0.150 mol) of 6-methyl isocytosine (manufactured by Tokyo Chemical Industry Co., Ltd.) and 130 mL of dimethyl sulfoxide were placed in a 200-mL four-necked flask equipped with a dropping funnel and a stirrer having a borosilicate glass rod with polytetrafluoroethylene (PTFE) stirrer blades under a nitrogen stream and heated to 150°C while mixing. While holding the mixture at 150°C, 25.6 g (0.165 mol) of isocyanate ethyl methacrylate (manufactured by Tokyo Chemical Industry Co., Ltd.) was added dropwise thereto in 30 minutes, and the mixture was held at 150°C for 30 minutes. The resulting reaction solution was cooled to room temperature and then mixed with 1300 mL of methanol to precipitate a white solid, which was collected by filtration. The resulting white solid was a compound represented by the following formula (I-I). Hereinafter, the compound (I-I) was used as an additive 1 for a rubber.
¹H-NMR: 1.93 (3H, 1 shown below), 2.23 (3H, 9 shown below), 3.58 (2H, 5 shown below), 4.27 (2H, 4 shown below), 5.55 (1H, 3 shown below), 5.78 (1H, 10 below), 6.18 (1H, 2 shown below), 10.50 (1H, 6 shown below), 11.95 (1H, 7 shown below), and 12.97 (1H, 8 shown below).

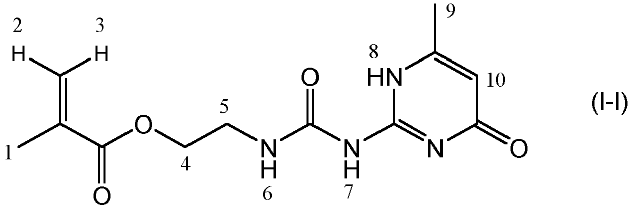

### Example 1-2 (Production of additive 2 for a rubber)

4.5 g (0.036 mol) of 6-methyl isocytosine (manufactured by Tokyo Chemical Industry Co., Ltd.) and 25 mL of dimethyl sulfoxide were placed in a 100-mL four-necked flask equipped with a dropping funnel and a stirrer having a borosilicate glass rod with polytetrafluoroethylene (PTFE) stirrer blades under a nitrogen stream and heated to 150°C while mixing. While holding the mixture at 150°C, 5.04 g (0.040 mol) of n-hexyl isocyanate (manufactured by Wako Pure Chemical Industries, Ltd.) was added dropwise thereto in 30 minutes, and the mixture was held at 150°C for 30 minutes. The resulting reaction solution was cooled to room temperature and then mixed with 250 mL of methanol to precipitate a white solid, which was collected by filtration. The resulting white solid was a compound represented by the following formula (I-II). Hereinafter, the compound (I-II) was used as an additive 2 for a rubber.
¹H-NMR: 0.87 (3H, 1 shown below), 1.30 (6H, 2 to 4 shown below), 1.63 (2H, 5 below), 2.22 (3H, 10 shown below), 3.23 (2H, 6 shown below), 5.81 (1H, 11 shown below), 10.12 (1H, 7 shown below), 12.84 (1H, 8 shown below), and 13.12 (1H, 9 shown below).

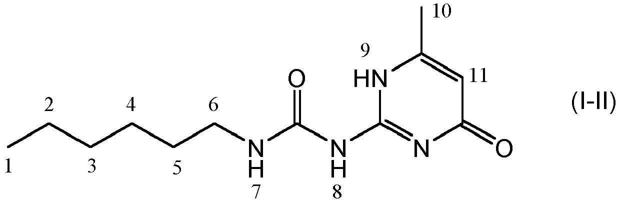

### Comparative Example 1-1 (Production of comparative additive 1)

With reference to JP 2014-221901 A, N-2-aminoethyl imidazolidinone was produced.

A 25% methanol solution of 165.0 g (1.6 mol) of diethylenetriamine (manufactured by Tokyo Chemical Industry Co., Ltd.), 45.45 g (0.5 mol) of dimethyl carbonate (manufactured by Wako Pure Chemical Industries, Ltd.), and 10.8 g (0.05 mol) of sodium methoxide (manufactured by Wako Pure Chemical Industries, Ltd.) was placed in a 300-mL four-necked flask equipped with a dropping funnel and a stirrer having a borosilicate glass rod with polytetrafluoroethylene (PTFE) stirrer blades under a nitrogen stream to be mixed at room temperature, and the mixture was held at 55°C for 1 hour. The temperature was raised to 90°C over a period of 30 minutes, held for 1 hour, and then further raised to 120°C over a period of 30 minutes and held for 2 hours. Thereafter, the methanol and excessive diethylene triamine were removed by vacuum distillation at 120°C, at 10 mmHg, so that a compound represented by the following formula (N-2-aminoethyl imidazolidinone) was obtained. Hereinafter, the compound was used as a comparative additive 1.

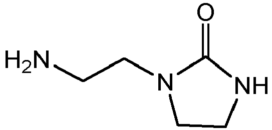

### Examples 2-1 to 2-4 and Comparative Examples 2-1 to 2-4 (Preparation of rubber composition and evaluation thereof)

Using a kneading extruder "LABO PLASTOMILL" (manufactured by Toyo Seiki Seisaku-sho, Ltd.), the components each shown in Table 1 and Table 2 were mixed at a mixing ratio shown in each of the Tables, and the mixture was kneaded through a first kneading step and a second kneading step in this order to prepare a rubber composition. The maximum temperature of the rubber composition in the first kneading step was set at 170°C, and the maximum temperature of the rubber composition in the second kneading step was set at 120°C. Vulcanization was performed at a temperature shown in the Tables, and the vulcanization time was specified as curelasto T90 value (min) × 1.5.

The curelasto T90 value (min) was obtained by measuring the time until the torque value reached 90% of the maximum torque (T90) using a curelastometer "FLAT DIE RHEOMETER MODEL VR-3110" manufactured by Ueshima Seisakusho Co., Ltd., when each of the rubber compositions was vulcanized at a temperature shown in the Tables.

The obtained rubber compositions were evaluated by the method described above. The results are shown in the Tables.

**Table 1**

| | | | | Example 2-1 | Example 2-2 | Comparative Example 2-1 | Comparative Example 2-2 |
|---|---|---|---|---|---|---|---|
| Compounding of rubber composition | First kneading step | SBR | Parts by mass | 100 | 100 | 100 | 100 |
| | | Carbon black | | 50 | 50 | 50 | 50 |
| | | Stearic acid | | 2 | 2 | 2 | 2 |
| | | Additive 1 for rubber | | 3 | 0 | 0 | 0 |
| | | Additive 2 for rubber | | 0 | 3 | 0 | 0 |
| | | Comparative additive 1 | | 0 | 0 | 0 | 3 |
| | Second kneading step | Zinc oxide | | 3 | 3 | 3 | 3 |
| | | Sulfur | | 1.2 | 1.2 | 1.2 | 1.2 |
| | | Vulcanization accelerator TBBS | | 0.5 | 0.5 | 0.5 | 0.5 |
| | | Vulcanization accelerator MBTS | | 0.5 | 0.5 | 0.5 | 0.5 |
| | | Vulcanization accelerator DPG | | 0.2 | 0.2 | 0.2 | 0.2 |
| Vulcanization temperature | | | °C | 160 | | | |
| Evaluation results | Storage modulus (dynamic strain: 0.1%) | | Index | 132 | 121 | 100 | 118 |
| | Storage modulus (dynamic strain: 10%) | | | 67 | 65 | 100 | 124 |
| | tan δ | | | 93 | 96 | 100 | 95 |

**Table 2**

| | | | | Example 2-3 | Example 2-4 | Comparative Example 2-3 | Comparative Example 2-4 |
|---|---|---|---|---|---|---|---|
| Compounding composition | First kneading step | SBR | Parts mass | 100 | 100 | 100 | 100 |
| | | Silica | | 50 | 50 | 50 | 50 |
| | | Coupling agent | | 5 | 5 | 5 | 5 |
| | | Stearic acid | | 2 | 2 | 2 | 2 |
| | | Additive 1 for rubber | | 5 | 0 | 0 | 0 |
| | | Additive 2 for rubber | | 0 | 5 | 0 | 0 |
| | | Comparative additive 1 | | 0 | 0 | 0 | 5 |
| | Second kneading step | Zinc oxide | | 3 | 3 | 3 | 3 |
| | | Sulfur | | 1.2 | 1.2 | 1.2 | 1.2 |
| | | Vulcanization accelerator TBBS | | 0.5 | 0.5 | 0.5 | 0.5 |
| | | Vulcanization accelerator MBTS | | 0.5 | 0.5 | 0.5 | 0.5 |
| | | Vulcanization accelerator DPG | | 0.2 | 0.2 | 0.2 | 0.2 |
| Vulcanization temperature | | | °C | 170 | | | |
| Evaluation results | Storage modulus (dynamic strain: 0.1%) | | Index | 134 | 101 | 100 | 83 |
| | Storage modulus (dynamic strain: 10%) | | | 91 | 93 | 100 | 91 |
| | tan δ | | | 86 | 87 | 100 | 84 |

The details of the components each shown in Table 1 and Table 2 are as follows.
SBR: a styrene-butadiene copolymer rubber, manufactured by Zeon Corporation, an emulsion polymerized SBR, trade name "NIPOL 1502";
Carbon black: manufactured by Tokai Carbon Co., Ltd., trade name "SEAST 3 (HAF)";
Silica: manufactured by Tosoh Silica Corporation, trade name "NIPSIL AQ";
Coupling agent: bis(3-triethoxysilylpropyl)tetrasulfide, manufactured by Evonik Industries AG, trade name "Si69";
Stearic acid: manufactured by Kao Corporation, trade name: "LUNAC S70-V";
Additive 1 for a rubber: a compound (I-I) produced in Example 1-1;
Additive 2 for a rubber: a compound (I-II) produced in Example 1-2;
Comparative additive 1: a compound (N-2-aminoethyl imidazolidinone) produced in Comparative Example 1-1;
Zinc oxide: manufactured by Wako Pure Chemical Industries, Ltd.;
Sulfur: manufactured by Wako Pure Chemical Industries, Ltd.;
Vulcanization accelerator TBBS: N-t-butyl-1,2-benzothiazolyl sulfenamide, manufactured by Wako Pure Chemical Industries, Ltd.;
Vulcanization accelerator MBTS: dibenzothiazyldisulfide, manufactured by Tokyo Chemical industry Co., Ltd.; and
Vulcanization accelerator DPG: 1,3-diphenyl guanidine, manufactured by Wako Pure Chemical Industries, Ltd.

### Industrial Applicability

Addition of the additive for a rubber of the present invention to a rubber composition allows the storage modulus at a small deformation to be improved without excessive improvement in the storage modulus at a large deformation, with tan δ being reduced. With use of the rubber compound containing the additive for a rubber in a tire, all of the fuel economy, the steering stability and the riding comfort of a vehicle can be satisfied.

## Claims

1. An additive for a rubber, comprising a compound represented by the following general formula (I): 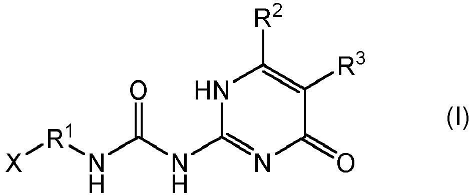 wherein R¹ is a divalent hydrocarbon group having 1 or more and 20 or less carbon atoms; R² and R³ are each independently a hydrogen atom or a hydrocarbon group having 1 or more and 6 or less carbon atoms; X is a hydrogen atom or a group represented by R⁴-COO-, R⁴-CONH- or R⁴-O-; and R⁴ is a hydrocarbon group having 1 or more and 20 or less carbon atoms.

2. The additive for a rubber according to claim 1, wherein in the general formula (I), R¹ is at least one selected from the group consisting of a straight chain or branched chain alkylene group and a straight chain or branched chain alkenylene group having 1 or more and 20 or less carbon atoms.

3. The additive for a rubber according to claim 1 or 2, wherein in the general formula (I), R² is an alkyl group having 1 or more and 6 or less carbon atoms and R³ is a hydrogen atom.

4. The additive for a rubber according to any one of claims 1 to 3, wherein in the general formula (I), X is a group represented by R⁴-COO-.

5. The additive for a rubber according to any one of claims 1 to 4, wherein R⁴ is a hydrocarbon group having 2 or more and 20 or less carbon atoms, having one or more polymerizable unsaturated bonds.

6. A rubber composition comprising an additive for a rubber according to any one of claims 1 to 5 and a rubber.

7. The rubber composition according to claim 6, further comprising a reinforcement filler.

8. A method for producing a rubber composition according to claim 6 or 7, comprising a step of compounding and kneading a compound represented by the general formula (I).

9. Use of a compound represented by the general formula (I) in producing a rubber composition according to claim 6 or 7.

10. A tire produced by using a rubber composition according to claim 6 or 7.

11. The tire according to claim 10, wherein the rubber composition is used in a tread part.

12. A method for producing a tire according to claim 10 or 11, comprising a step of molding the rubber composition.

13. Use of a rubber composition according to claim 6 or 7 in a tire.

14. Use of a rubber composition according to claim 6 or 7 in a tread part of a tire.
